# EUROPEAN PATENT SPECIFICATION

(11) **EP 0 994 726 B1**
(45) Date of publication and mention of the grant of the patent: **17.04.2002**
(21) Application number: 97930669.3
(22) Date of filing: 10.07.1997
(51) Int. Cl.: A61K 47/48

(54) **COMPOSITIONS SUITABLE FOR DELIVERY OF GENES TO EPITHELIAL CELLS**
ZUSAMMENSETZUNGEN ZUR EINFÜHRUNG VON GENEN IN EPITHELZELLEN
COMPOSITIONS CONVENANT A LA DISTRIBUTION DE GENES DANS DES CELLULES EPITHELIALES

(30) Priority: 10.07.1996 GB 9614441; 10.07.1996 GB 9614471
(43) Date of publication of application: 26.04.2000
(73) Proprietor: West Pharmaceutical Services Drug Delivery & Clinical Research Centre Limited, Nottingham NG7 2TN (GB)
(72) Inventor: ILLUM, Lisbeth, Nottingham NG7 1BA (GB)
(74) Representative: McNeeney, Stephen Phillip
(86) International application number: GB9701859
(87) International publication number: WO9801160

(56) References cited:
- WO-A-96/00295
- WO-A-96/05810
- WO-A-96/40264
- US-A- 4 046 750
- CHEMICAL ABSTRACTS, vol. 126, no. 12, 24 March 1997 Columbus, Ohio, US; abstract no. 153349, MURATA, JUN-ICHI ET AL: "The molecular design of quaternary chitosan conjugate for gene delivery having recognition ability" XP002048851 & KICHIN, KITOSAN KENKYU (1996), 2(2), 158-159 CODEN: KKKEFB;ISSN: 1340-9778, 1996,
- MURATA J-I ET AL: "Possibility of application of quaternary chitosan having pendant galactose residues as gene delivery tool." CARBOHYDRATE POLYMERS 29 (1). 1996. 69-74. ISSN: 0144-8617, XP002048848
- DATABASE WPI Section Ch, Week 9141 Derwent Publications Ltd., London, GB; Class B04, AN 91-299437 XP002048853 & JP 03 198 782 A (KURITA WATE IND KK) , 29 August 1991
- MAO, H.-Q. ET AL: "DNA-chitosan nanospheres: derivatization and storage stability" PROC. INT. SYMP. CONTROLLED RELEASE BIOACT. MATER. (1997), 24TH, 671-672 CODEN: PCRMEY;ISSN: 1022-0178, 1997, XP002048849
- ROY, KRISHNENDU ET AL: "DNA-chitosan nanospheres: transfection efficiency and cellular uptake" PROC. INT. SYMP. CONTROLLED RELEASE BIOACT. MATER. (1997), 24TH, 673-674 CODEN: PCRMEY;ISSN: 1022-0178, 1997, XP002048850
- MUMPER, R. J. ET AL: "Novel polymeric condensing carriers for gene delivery" PROC. INT. SYMP. CONTROLLED RELEASE BIOACT. MATER. (1995), 22ND, 178-9 CODEN: PCRMEY;ISSN: 1022-0178, 1995, XP002048876
- Mao, J-I. et al.: PROCEED. INTERN. SYMP. CONTROL. REL. BIOACT. MATER., 23, 1996, pp. 401-402

## Description

The present invention relates to the use of a non-viral vector based on a cationic polymer, chitosan (polyglucosamine).

Gene therapy represents an exciting opportunity in medical treatment. The insertion of an appropriate gene into cells *in vitro* and *in vivo* could be beneficial in the treatment of genetic diseases as well as a means of producing complex drugs in the body. A variety of applications for gene therapy has been described in reviews of the subject (see for example Evans *et al*, (1995) *British Medical Bulletin* **51**, 226-234 and Wolff (Editor) Gene Therapeutics, *Methods and Applications of Direct Gene Transfer.* Birkhauser, 1994).

A major problem in gene therapy is the effective delivery of the DNA construct to the target organ and for the DNA to be taken up into the appropriate cells so that expression can result. Indeed the need for new delivery systems has been identified as a major issue in successful gene therapy (see for example Wilson (1993) *Nature* ***365,*** 691, Ledley (1994) *Current Opinion in Biotechnology,* **5** 626-636). Such gene delivery systems are often termed vectors. These vectors can be divided into two main types; viral and non-viral (see for example Schofield and Caskey (1995) *British Medical Bulletin* **51,** 56-71).

A wide variety of non-viral vectors have been described in the prior art. These include monocationic lipids such as DOTMA (N[1-(2,3-dioleoyloxy)propyl]*-N,N,N*-trimethylammonium chloride as well as cationic polymers. The best known polymer for gene delivery is polylysine but this is known to be toxic. Other cationic polymers to include complex structures such as dendrimers have been described (Haensler and Szoka (1993) *Bioconjugate Chem* **4,** 372-379).

Gene therapy models of enhanced anriinflammatory peptide expression for the treatment of intestinal inflammation including inflammatory bowel disease have been described (Pizarro, TI (1996); *IBC Conference of Inflammatory Bowel Disease,* June 3, 1996, Philadelphia USA). The use of gene therapy in gastroenterology has been reviewed by Dandha and Lemoine (1996) *Gut* **38**, 141-145. The prior art contains various descriptions of methods for the delivery of gene products to the gastrointestinal tract using non-viral vectors. These have included liposome systems given by rectal infusion (Westbrook *et al*, (1994) *Human Molecular Genetics*, **3**, 2005-2010) and slow release capsules (WO93/19660).

In general the levels of expression in delivering DNA to epithelial surfaces have been low and in some cases the results have been confounded by the use of an inappropriate reporter gene. For example, the expression of beta-galactosidase in the gastrointestinal tract can be greatly affected by endogenous levels of the enzyme and other factors that will lead to false positive results. The marker CAT (chloramphenicol acetyl transferase) is considered to be a good reporter of expression because of its bacterial origins.

We have found, surprisingly, that it is possible to obtain enhanced levels of expression in the epithelial tissues of a model animal (the rabbit) by the complexation of DNA with the material chitosan (polyglucosamine).

Chitosan has been described in our previous patent applications as a novel material for enhancing the uptake of peptides and small molecular weight polar molecules across biological membranes leading to an improved therapeutic effect (see also Illum *et al*, (1994) *Pharmaceutical Research* **11,** 1186-1189, Artursson *et al,* (1994) *Pharmaceutic Research* **11,** 1358-1361). Complexes between chitosan and therapeutic agents such as polypeptide drugs have also been described (PCT/GB90/00291). Complexes between chitosan and DNA have been described for use in delivering DNA to cells for the expression of polypeptides to serve as antigens (GB96/03019) or as a drug (WO96/05810). However, the levels of expression achieved were not fully characterised and the complexes were not optimised for the delivery and expression of DNA. The examples described in WO96/05810 were prepared by spray drying, emulsification, solvent evaporation, or precipitation by exposure to alkaline conditions, with or without crosslinking. Methods of preparing microparticles comprising chitosan and DNA are not described in detail in GB96/03019.

In summary, these prior art methods could not be used to prepare compositions comprising an integral mixture of DNA and chitosan which are in the form of nanoparticles, and can thus be taken up easily by cells, such as epithelial cells.

The condensation of plasmid DNA by cationic polymers, including chitosans of different molecular weights has been described by Mumper *et al* in Proc. Intern. Symp. Control. Rel. Bioact. Mater., **22**, 325 (1995). These studies were *in vitro* investigations directed to the interaction of compacted DNA with muscle cells. The physicochemical evaluation of chitosan-DNA complexes is described, as is the weak *in* *vitro* transfection of a muscle (myocyte) cell-line. No *in vivo* data were presented. More importantly, that chitosan:DNA complexes would be of particular benefit for the transfection of epithelial cells, and more particularly for the transfection of cells and structures that play an important role in the mucosal immune response, is not mentioned or suggested.

JP-A-3198782 describes a complex of a gene and a carrier which is composed of low molecular chitosan. This complex can be used to introduce the gene to a cell.

The formation of a polyelectrolyte complex of an N,N,N-trimethylchitosan/galactose. conjugate and DNA is reported in Murata, J-I *et al*, Carbohydrate Polymers, vol. 29, no. 1, 1996, pages 69-74. The use of this complex as a gene delivery tool was also investigated.

A nanosphere delivery vehicle made by the complex coacervation of DNA and chitosan is described in Mao, H-Q, Proceed. Intern, Symp. Control. Rel. Bioact. Mater., 23^{rd}, 1996, pages 401 and 402.

WO98/01162 describes a gene delivery system made of enzymatically degradable polymeric cation and nucleic acid nanospheres optionally with a linking moiety or a targeting ligand attached to the surface.

WO97/42975 describes compositions of chitosan-based compounds and nucleic acids or oligonucleotides which are capable of delivery to a cell.

The present invention provides the use of a composition comprising a particulate complex of chitosan and nucleic acid wherein the complex is between 10 nm and 1µm in size and carries a surface charge in the manufacture of a medicament for the delivery of said nucleic acid to an epithelial surface of a mammal, wherein the nucleic acid is capable of being expressed and encodes an antiinflammatory agent, an antiinfective agent, sucrose-isomaltase, lactase-phlorizin hydrolase, maltase-glucoamylase, a drug or an antisense agent for the treatment of colon cancer.

Microspheres comprising nucleic acid and chitosan are prepared by mixing the nucleic acid and chitosan together, as described hereinafter, without the need for further treatment. Chitosan is believed to compact the plasmid DNA into a nanoparticle of between 10 nm and 1µm and to confer upon the compacted material suitable surface characteristics that will lead to good adherence of the particle to the surface of the target cell followed by internalisation that will lead to expression of the encoded material.

We describe herein the use of chitosan complexes as non-viral vectors to enhance the expression of nucleic acid in epithelial tissues, such as the gastrointestinal tract, the vagina, the rectum, the nasal cavity, the lungs and the buccal cavity. By "nucleic acid" we means nucleic acids, particularly DNA, including oligonucleotides greater than 100 base pairs, plasmids or cosmids thereof, coiled or uncoiled.

We have also found surprisingly that the chitosan complexes described herein enhance the expression of nucleic acid in mesenteric lymph nodes in the gastrointestinal tract and draining lymph nodes in the nose.

The compositions used according to the present invention comprise a particulate complex of chitosan and a nucleic acid wherein the complex is between 10 nm and 1 µm in size and carries a surface charge wherein the nucleic acid is capable of being expressed and encodes an antiinflammatory agent, an antiinfective agent, sucrose-isomaltase, lactase-phlorizin hydrolase, maltase-glucoamylse, a drug or an antisense agent for the treatment of colon cancer.

It is preferred that, when the nucleic acid is DNA, the surface charge is above +10 mV for DNA:chitosan ratios between 1:1 and 1:6 and above +20 mV for all other DNA:chitosan ratios.

The size of the nucleic acid-chitosan complex can be between 10 nm and 1 µm. preferably between 20 nm and 700 nm, and most preferably between 50 nm and 500 nm (hydrodynamic diameter, as measured by Photon Correlation Spectroscopy).

By the term "chitosan" we mean polyglucosamine and oligomers of glucosamine materials of different molecular weights and degrees of acetylation. Modified chitosans, particularly those conjugated to polyethylene glycol, are included in this definition.

Low and medium viscosity chitosan (for example CL113, G210 and CL110) may be obtained from various sources, including Pronova Biopolymer, Ltd., UK; Seigagaku America Inc., MD, USA; Meron (India) Pvt, Ltd., India; Vanson Ltd, VA, USA; and AMS Biotechnology Ltd., UK.

The chitosan-nucleic acid complexes described may be prepared by the following method. The method comprises mixing the two components together, the rate of mixing, the relative concentrations of the starting solution, the ratios of the two components, pH, ionic strength and temperature being such as to achieve, non-inventively, the required particle size and charge. Particle size and charge can be easily determined using techniques known to those skilled in the art such as those described hereinafter. The rate of mixing, the relative concentrations of the starting solution, pH, ionic strength and temperature can be altered, for example as described hereinafter, as part of a standard experimental design to control particle size. The ratios of the two components can also be used to control size and surface charge. The weight to weight ratio between nucleic acid and chitosan can be between 1:1 and 1:25, preferably between 1:2 and 1:20 and most preferably between 1:5 and 1:6.

The size and size distribution of the nucleic acid-chitosan complexes may be determined by Photon Correlation Spectroscopy (PCS) using a Malvern 4700 submicron particle analyser system (Malvern Instruments, UK) with a PCS 100 spectrometer. The samples are diluted with distilled water and measured at 25°C at a scattering angle of 90°. The size distribution is characterised by a polydispersity index (PI).

The molecular weight of the chitosan influences the size of the compacted nucleic acid complex, and indeed can be chosen so as to influence the size of the complex. To this end, studies can be conducted using ethidium bromide fluorescence as described by Wolfert and Seymour (1996) *Gene Therapy* 3, 269-273. Ethidium bromide interacts with nucleic acid to provide strong fluorescence. When a complexing cationic polymer is added the ethidium bromide is squeezed out of the complex and the fluorescence reduced. A plot of fluorescence against quantity of added polymer can be used to assess the compaction process (loss of fluorescence is indicative of degree of compaction) and thereby the selection of the appropriate molecular weight of the complexing chitosan species. Fluorescence may be measured using Perkin-Elmer 3000 Fluorescence Spectrometer, for example. The optical emission wavelength is set at 591 nm and optical excitation wavelength is set at 366 nm. Ethidium bromide (EtBr) is added to water in a cuvette (reference) and nucleic acid solution and ethidium bromide are added to water in a second cuvette (test). The fluorescence of the reference cuvette is set to zero. The fluorescence obtained for the test cuvette is measured. Chitosan samples are then introduced in very small portions to the test cuvette with constant stirring, and the fluorescence reading is taken three times, returning to the zero cell in between each reading. The average of the three is then recorded.

The molecular weight of the chitosan is preferably between 500 Dalton and 500,000 Dalton, more preferably 700 Dalton to 250,000 Dalton and most preferably 1,000 Dalton to 150,000 Dalton.

Chitosans of different low molecular weight can be prepared by enzymatic degradation of chitosan using chitosanase or by the addition of nitrous acid. Both procedures are well known to those skilled in the art and are described in recent publications (Li *et al,* (1995) *Plant Physiol. Biochem.* **33,** 599-603; Allan and Peyron, (1995) *Carbohydrate Research* **277,** 257-272; Damard and Cartier, (1989) *Int. J. Biol. Macromol.* **11,** 297-302).

It is preferred that the complex has a surface charge larger than +1 mV. More preferably, the surface charge is between +2 mV and +100 mV, more preferably between +5 mV and +60 mV, and particularly between +20 mV and +60 mV.

The surface charge of the particles is expressed as a zeta potential. This is determined at pH 7.4 and 0.01 M ionic strength. The zeta potential is measured by Laser Doppler Anemometry (LDA) using for example a Malvern Zeta Sizer IV (Malvern, UK). Laser Doppler Anemometry involves the detection of scattered laser light from particles which are moving in an applied electric field. The equipment used can for example be a Malvern Zetasiser II (Malvern Instruments).

Electrophoretic mobility measurements are carried out by dispersing tile nucleic acid-chitosan complexes in 10 ml buffer solution such as phosphate or McIlvaine buffer, from 100 µg - 1 mg, with a constant ionic strength 0.01M. Four readings are taken using a PC4 wide capillary cell at a voltage of 100 volt, electrode spacing of 50 mm and a dielectric constant of 78.54.

The nucleic acid may be any nucleic acid. Preferably it is DNA. More preferably it is DNA oligonucleotides greater than 100 base pairs, or plasmids or cosmids, which may be coiled or uncoiled. The nucleic acid is capable of being expressed. For example, it may be a DNA expression vector, such as a plasmid, containing an insert in proper orientation and correct reading frame for expression by the desired host cells. An example of such a DNA is the reporter gene plasmid pCAT-DNA driven by a CMV promoter, supplied by GeneMedicine, Houston, USA.

The nucleic acid may encode a gene product which may act as a therapeutic agent for an inherited or acquired disease, which may include therapeutic agents for treatment of local conditions such as Crohn's disease, ulcerative colitis, asthma, cystic fibrosis and cancer.

Examples include antiinfective agents, antiinflammatory agents, and prophylactic agents. Gene products include but are not limited to alpha 1-antitrypsin, growth hormone synthase, factor VII, factor IX, TNF, cytokines, antigen genes (hepatitis B, influenza, cholera), the cystic fibrosis transmembrane conductance regulator CTFR, cancer genes (p53, K-ras, HS-Ek), sucrose-isomaltase, lactase-phlorizin hydrolase and maltase-glucoamylase.

The nucleic acid-chitosan formulations described herein may be delivered to endothelial surfaces or into tissues, particularly the muscles or into body compartments such as the joints and lung (by aerosolisation). They may be applied to epithelial surfaces such as those of the nasal cavity, lungs (including any region of the lung), vagina, uterus or the different regions of the gastrointestinal tract (buccal cavity, oesophagus, stomach, small intestine, large intestine (colon), the gut associated lymphoid tissue (Peyer's patches and colonic lymphatic follicles) or rectum). In particular the epithelial surface may be the nasal mucosa, the nose associated lymphoid tissue (to include the tonsils and Waldayer's ring in man). Further, the epithelial surface may be the eye (i.e. the corneal surface of the eye) or the inner ear.

The particles may be delivered using appropriate administration devices familiar to those skilled in the art. For example, commercially available liquid and powder spray systems can be used for nasal administration, for example liquid systems such the nasal metered dose spray (Pfeiffer) and the Monospray - nasal liquid unidose (Valois); or powder systems such as the Monopoudre (Valois), the Lyphodose (Valois), the Nasalet (Fisons), the single dose insufflator (Miat) the Turbuspin (PH&T) and the Unit Dose System (Bespak). Aerosol systems can be used for administration to the lungs (to include propellant devices, dry powder systems, nebulizers (Venturi and ultrasonic as well as extrusion systems)). For administration to the vagina the chitosan-complex formulation can be formulated into a gel and administered using a syringe type device. For the rectum the chitosan-complex can be administered formulated into a suppository or as a suspension administered as an enema. The powder formulations could include systems where the compacted material is formulated into or onto a microsphere prepared from a biocompatible and biodegradable material such as a polysaccharide, starch or modification thereof, albumin or gelatin.

Formulations can be administered as immediate or controlled release systems. Systems that gel upon contact with the mucosa or undergo gelling due to a change in environmental conditions are considered to be particularly relevant. Examples include but are not limited to gellan gum, xanthan, alginate, synergistic polymers. Site specific delivery could be achieved using delivery systems known to those skilled in the art. For example, formulations that deliver drugs specifically to the colon have been described in our patent applications WO91/11175 and WO95/35100.

The efficiency of cell uptake may be enhanced by the co-administration of a mucolytic agent such as tyloxapol, N-acetylcysteine (to allow better access to the epithelial cells) as well as by the addition of coating agents that will exploit various lectin mediated processes known in the gastrointestinal tract (e.g. fimbrial adhesins, tomato lectin). Invasins represent a special class of added materials that may aid the adhesion and uptake of the complex particles into epithelial cells (see, for example, A. T. Florence, Pharm. Res., **14**, 259 (1997).

Greater retention at an epithelial surface may be afforded if the complex is bound to the surface of a larger microsphere. The term "microsphere" is used herein to include both solid matrix microspheres and microcapsules. Biodegradable microspheres that are of the appropriate size and surface charge density can be formulated from a number of different materials, such as soluble starch, carrying carboxyl groups covalently linked to the starch, and polymers that contain groups that ionize to allow electrostatic interaction between positively charged DNA conjugates and the biodegradable microspheres. Such groups are preferably carboxyl or sulphate groups. Such polymeric materials include the alginates, heparin and other sulphated polysaccharides (carboxymethyl dextran, carboxydextran and dextran sulphate) and biodegradable synthetic polymers such as polylactide coglyceride and polylactic acid. Likewise, a physical mixture of polymers that is then converted into microspheres may be used. For example, heparin/albumin microspheres have previously been described for the purposes of drug delivery (Kwon *et al*, (1991) *J. Colloid Interface Sci.* **143**, 501 and Cremers *et al*, (1990) *J. Controlled Release* **11**, 167. Such materials have not been described for use in gene therapy nor for the carriage of DNA conjugates.

Hence, the biodegradable microspheres may be prepared from crosslinked soluble starch, albumin, gelatin, heparin, carboxydextran, dextran, dextran sulphate, alginate, polylactide, or their admixtures.

The biodegradable microspheres should preferably have an average size (mean number diameter, as determined by light microscopy) larger than 5 µm, more preferably between 6 and 200 µm, most preferably between 10 and 100 µm and still more preferably between 12-50 µm. The size of the biodegradable microspheres described herein refers to the size of the swollen particle in the vehicle chosen for intravenous administration.

The properties of the carrier system can be so designed as to provide different rates of degradation and thereby control the period of contact between the carrier microsphere and the epithelial surface. The different rates of degradation can be achieved by methods known to those skilled in the art such as regulating the degree of crosslinking of the microspheres; the less crosslinked microspheres degrade faster than the microspheres which are crosslinked to a higher degree. Likewise for some polymers it is possible to control the rate of degradation by selection of lower molecular weight polymer chains for faster degradation and higher molecular weight polymer chains for slower degradation. For systems that are crosslinked by heating, the degree of crosslinking and therefore the rate of degradation can be controlled by the period of heating and the temperature.

Suitable microparticles for use in the compositions are commercially available and include starch microspheres (such as Cadoxamer (Perstorp) and Spherex (Pharmacia Upjohn)), dextran microspheres (CM Sephadex (Pharmacia Upjohn) and SP Sephadex (Pharmacia Upjohn)) and agarose microspheres (CM Sepharose (Pharmacia Upjohn) and S Sepharose (Pharmacia Upjohn)). Albumin and gelatin microspheres may be prepared according to methods which are well known to those skilled in the art.

An example of the administration of DNA-chitosan formulations to the gastrointestinal tract of rabbits is given. It involves laparotomy (surgical incision in the abdomen) and recovery of the rabbits. Prior to each study day fur is removed from the abdominal area of the appropriate rabbit using hair clippers. On each study day, the appropriate rabbits are anaesthetised using a Boyles Apparatus / halothane anaesthesia. Anaesthesia is maintained through the procedure. A midline incision of between 5 - 8 cm is made through the skin to expose the abdomen. A second midline incision of between 5 - 8 cm is made through the white-line (to minimise operative bleeding) running longitudinally down the abdomen of the rabbit to access the abdominal cavity. The intestines are exposed and the colon and upper part of the small intestine are identified with minimal handling of the intestines. It is necessary to exteriorise part of the intestines to gain clear access to the small intestine and colon for dose administration.

For administration to the small intestine, the dose is administered to the upper part of the small intestine approximately 10 cm from the junction with the stomach. For administration to the colon, the dose is administered to the colon approximately 5 cm from the junction with the caecum.

For dose administration, the appropriate dose volume is administered directly into the lumen of the appropriate part of the gastrointestinal tract using a sterile syringe and needle. On withdrawal of the needle the tissue is sealed with a drop of cyanoacrylate adhesive to prevent dose leakage. Care is taken to ensure that the cyanoacrylate adhesive dries before the exteriorised intestines are replaced in the abdominal cavity and that the intestines lie in approximately their natural position. The abdominal cavity is closed with two layers of sutures, one through the abdominal musculature and the second through the skin. The rabbits are returned to the appropriate housing room and allowed to recover from the effects of the anaesthetic.

Further examples are delivery of DNA to the lungs to produce cystic fibrosis transmembrane conductance regulator (CFTR) for treatment of cystic fibrosis, or delivery of DNA to the nasal or vaginal cavities to produce therapeutic agents for treatment of local-conditions such as inflammation, for the treatment of allergies or HIV, or agents for use as contraceptives.

A further aspect of the invention is the use of a composition of the invention in medicine.

An aspect of the invention is the use of a composition as described herein wherein the nucleic acid encodes sucrose-isomaltase, lactase-phlorizin hydrolase or maltase-glucoamylase in the preparation of a medicament for use in the treatment of disaccharide intolerance.

Another aspect of the invention is the use of a composition as described herein wherein the DNA encodes a drug or antisense agent in the manufacture of a medicine for the treatment of colon cancer.

The present invention will now be described in more detail with reference to the following figures and examples.

Figure 1: Calibrated heat flow graph of pCAT-DNA titrated with chitosan.

Figure 2: CAT concentration of cell extracts from C6 cell line transfected with pCAT DNA from various chitosan formulations (mean ±SD).

Figure 3: CAT concentration in tissue extracts from different regions of the gastrointestinal tract of rabbits.
Sampled tissue: 1 PP1 ; 2 PP2 ; 3 PP3 ; 4 EN1 ; 5 EN2 ; 6 EN3 ; 7 COL

### Example 1: CAT analysis

### Materials

The reporter gene pCAT-DNA driven by a CMV promoter was supplied by GeneMedicine, Houston, USA. ApCAT-DOTMA (N[1-(2,3-dioleoyloxy)-propyl]-NNN-trimethylammonium chloride) complex was used as a control material. This was prepared as outlined by Felgner *et al*, (1987), *Proc. Natl. Acad. Sci. (UA)* **84,** 7415-7417.

### Methods

A control pCAT DNA solution was prepared by the dilution of the pCAT-DNA stock solution (0.811 mg/ml) in autoclaved PBS. To 0.863 ml of the pCAT-DNA stock solution were added 13.137 ml of autoclaved PBS. This provided 14.0 ml of control pCAT-DNA solution (0.05 mg/ml), sufficient to dose six rabbits (3 into small intestine and 3 into colon) with 100 µg of pCAT-DNA in a volume of 2 ml. The solution was stored at 4°C and warmed to room temperature 15 min prior to dosing the rabbits.

A pCAT-DNA DOTMA formulation supplied in the form of lyophilised powder was reconstituted on the appropriate study day using sterile water. The resultant liquid formulations contained a sufficient concentration of plasmid DNA to administer a dose of 100 µg of pCAT-DNA in a volume of 2 ml. The formulation was stored at 4°C and warmed to room temperature 15 min prior to dosing the rabbits.

### CAT ELISA

The CAT ELISA procedure for the analysis of CAT in the cell transfection samples and the animal tissues was set up following the protocol supplied by the manufacturer of the kit (Boerhinger Mannheim). The assay is based on the sandwich ELISA principle, where the anti-CAT antibody is prebound to the surface of the microtitre plate modules, the CAT enzyme in the extracts of the transfected cells and the tissues, binds specifically to the immobilised anti-CAT antibody and digoxogenin-labeled antibody to CAT (anti-CAT-DIG) binds to CAT enzyme. The detection antibody, peroxidase conjugated anti-digoxigenin antibody (anti-DIG-POD) binds to digoxigenin. In the final step the peroxidase substrate (ABTS; 2,2'-azinobis[3-ethylbenzthiazoline sulphonic acid]) is catalysed by peroxidase yielding a colour reaction product which is enhanced by the use of a substrate enhancer. The absorbance of this colour is then directly correlated to the amount of CAT enzyme present in the standards and the extracts.

### Validation of CAT ELISA

Prior to analysing the test samples, the CAT ELISA procedure was validated. Since the appropriate quality control tissue samples containing CAT were not available, both the infra-day and inter-day variations were determined using the standard curves. These curves were prepared using the CAT standards supplied in the CAT ELISA kit at CAT concentrations of 0.0, 15.6, 31.25, 62.5, 125, 250 and 500 pg/ml. The intra-day variation was determined by preparing the standard curve in triplicate. Both for the intra-day and inter-day variation the coefficient of variation (%CV) at each standard concentration was calculated.

### Validation of the tissue extraction procedures

The tissue extraction procedure was initially established by taking the various tissue samples from two control rabbits dosed with "naked" plasmid DNA either in the small intestine or in the colon and dividing them into three sections and storing separately at -80°C. 0.5 ml of ice-cold tissue extraction buffer (10 mM Tris pH 7 - 8; 150 mM NaCl, 1 mM EDTA, 0.5% Triton X- 100, 100 µM Leupeptin, 1.0 µM pepstatin and 0.25 mM PMSF) was added to the tube containing the tissue sample and homogenised four different times (0.6, 1.2 or 2.4 min) using a Mini-Bead Beater at 4°C. The homogenate was transferred to a sterilised microfuge tube (1.5 ml) and centrifuged in an MSE Microfuge at 13,000 rpm for 20 min at 4°C. The supernatant (i.e. tissue extract) was transferred to a sterilised, microfuge tube (1.5 ml) and placed on ice. An aliquot of the tissue extract was diluted (1 in 20) with PBS and assayed for protein concentration using the BCA protein assay. Each sample was analysed in duplicate and corrected for dilution in order to obtain the total protein concentration (mg/ml) in the tissue extract.

The CAT ELISA procedure was validated for the analysis of CAT in the gastrointestinal tissue extracts. Firstly, the effect of protein concentration on the determination of CAT concentration in the various tissue extracts using the CAT ELISA procedure was assessed. Tissue samples from the control rabbits (Peyer's patches, enterocytes, colonic tissue and mesenteric lymph nodes) were extracted in ice-cold tissue extraction buffer (0.5 ml) as described above but were homogenised for 1.5 min (3 cycles of 30 sec. homogenisation and 30 sec. rest). After determining the protein concentration, the tissue extracts were diluted with Sample Buffer (CAT ELISA kit) to produce extract solutions with protein concentrations of approximately 0.5, 1.0 and 2.0 mg/ml). Each of these diluted extracts were spiked with CAT standard (CAT ELISA kit) to given final CAT concentrations of 25 and 100 pg/ml. The spiked samples were then analysed in duplicate for CAT concentration and the percentage recovery of CAT in each sample was calculated.

Secondly, the effect of extraction procedure on the stability of CAT in the various tissues was established. This was carried out by extracting the tissue samples from the above control rabbits (colonic tissue, Peyer's patch and enterocytes) with extraction buffer containing CAT concentrations of 50 or 200 pg/ml. After determining the protein concentrations each sample extract was diluted (1 in 2 or 1 in 4) with the Sample Buffer. Each diluted sample was analysed in duplicate for CAT concentration. After correcting for dilution the percentage recovery of CAT in each tissue extract was calculated.

### Analysis of transfected cells

Cell extracts of the transfected cells (RAW 264 macrophage and C6 glial) were prepared using the freeze/thaw method. The cells were scraped off the surface of the plate and transferred to a sterile microfuge tube and were rinsed well with 400 µl of pre-cooled TEN buffer (40 mM Tris-HCl, 1 mM EDTA, 150 mM sodium chloride, pH 7.8). The cells spun down in an MSE Microfuge for 1 min at maximum speed (13,000 rpm). After removing the supernatant the cells were resuspended in 150 µl Tris buffer (250 mM Tris-HCl, pH 7.8). The cells were frozen in liquid nitrogen for 5 min and then thawed at 37°C in a water bath for 5 min. This freeze/ thaw cycle was repeated 4 times. The cell suspension was centrifuged in an MSE Microfuge for 10 min at maximum speed (13,000 rpm). The cell extract (supernatant) was transferred to fresh microfuge tube. An aliquot of the supernatant was analysed for protein concentration using the BCA Protein Assay. The cell extracts were diluted (1 in 5) with Sample Buffer (CAT ELISA kit) containing 0.5 mM EDTA and 8 µg/ml of Trasylol and stored at -80°C prior to CAT analysis. Each cell extract dilution was analysed in duplicate for determining CAT concentrations. The values less than 15 pg/ml were assumed as zero and the values higher than this concentration were appropriately corrected for dilution in order to obtain the total CAT concentration (pg/ml) in the cell extract. Since the total protein concentration in each cell extract was different, the amount of CAT expression in each sample was normalised by dividing the total CAT concentration by the total protein concentration

### Analysis of rabbit tissue samples

The extracts of the rabbit tissues were prepared before analysing for CAT. Ice-cold tissue extraction buffer (1 ml) was added to the tube containing the sample and homogenised using a Mini-Bead Beater at 4°C for 1.5 min (3 cycles of 30 sec homogenisation and 30 sec rest). The homogenate was transferred to a sterilised microfuge tube (1.5 ml) and centrifuged in an MSE Microfuge at 13,000 rpm for 20 min at 4°C. The supernatant (i.e. tissue extract) was transferred to a sterilised, microfuge tube (1.5 ml) and placed on ice. An aliquot of the tissue extract was diluted (1 in 20) with PBS and assayed immediately for protein concentration using the BCA protein assay. Each sample was analysed in duplicate and corrected for dilution in order to obtain the total protein concentration (mg/ml) in the tissue extract.

An aliquot of the tissue extract was appropriately diluted using the Sample Buffer (1 in 2, 1 in 4 or 1 in 3, 1 in 6) and stored at -80°C prior to CAT analysis. Each tissue extract dilution was analysed in duplicate for determining CAT concentration. The values less than 15 pg/ml were assumed as zero and the values higher than this concentration were appropriate corrected for dilution in order to obtain the total CAT concentration (pg/ml) in the tissue extract. Since the total protein concentration in each tissue extract was different, the amount of CAT expression in each tissue was normalised by dividing the total CAT concentration by the total protein concentration.

For intra-day variation the CV values for all the standards were within acceptable limits (±10%). For inter-day variation although the CV values were within acceptable limits (±20%) only for standards at the upper end of the curve (125 - 500 pg/ml), the mean correlation coefficient of the standard curves was 0.996 ± 0.002. The observed inter-day variability in the standard curve was possibly due to slight changes in the incubation time with the substrate in the last step of the assay and also due to plate variation from different kits. The lowest detectable concentration of CAT was 15 pg/ml and the upper limit of the assay was 500 pg/ml.

Each tissue sample had similar protein concentrations after extracting for different times (0.6, 1.2, 2.4 min), thereby showing that the samples could be satisfactorily homogenised within 2.4 min.

The recovery of CAT from spiked tissue extracts at protein concentrations of 0.5, 1.0 and 2.0 mg/ml was 83 to 100%. The recovery of CAT from tissues spiked with CAT prior to extraction varied in different tissue types and were 50% or less when the homogenates had protein concentrations of 4 mg/ml or higher.
Therefore, a high protein concentration could cause under estimation of CAT expression in a test sample. All subsequent determinations were made with this result in mind.

### Example 2

### Preparation of Chitosan complexes

Chitosan CL113 was provided by Pronova Biopolymer, Ltd., UK. pCAT-DNA chitosan complexes with ratios of 1:10 and 1:5 (w/w) were prepared according to the method described in Example 7 below by the slow addition of chitosan solution to pCAT-DNA solution with constant stirring. The sizes of the resultant particles were 100 - 200 nm (S) and 300 - 460 nm (L). The size of the particles was determined by the rate at which chitosan was added to DNA solution; the faster the rate, the smaller the particles. The preparation with a ratio of 1:10 and particle size of 300 - 460 nm was selected for evaluation in the animal studies. The concentration of pCAT DNA in the formulation was 38.677 µg/ml.

The size and size distribution of the pCAT-DNA chitosan complexes was determined by Photon Correlation Spectroscopy (PCS) using a Malvern 4700 submicron particle analyser system (Malvern Instruments, UK) with a PCS 100 spectrometer. The samples were diluted with distilled water and measured at 25°C at a scattering angle of 90°. The size distribution was characterised by a polydispersity index (PI) (Table 1).

**Table 1:**

| **Characteristics of pCAT chitosan complexes** | | | | |
|---|---|---|---|---|
| Batch No | pCAT:chitosan ¹ | Actual size (nm) | PI ² | Mean Zeta potential (mV) |
| 1 | 1:5, Small | 138.3 | 0.186 | 28.7 |
| 2 | 1:5, Large | 302.2 | 0.483 | 30.1 |
| 3 | 1:10 Small | 172.5 | 0.214 | 39.4 |
| 4 | 1:10 Large | 463.9 | 0.543 | 39.6 |

| | | | | |
|---|---|---|---|---|
| 1. As defined by particle size measurement, Small 100 - 200 nm, Large 300 - 500nm | | | | |
| 2. Polydispersity index | | | | |

The zeta potential of pCAT-DNA chitosan complexes was determined using the technique of electrophoretic laser Doppler spectrometry. The medium for the zeta potential measurement was 1 mM M pH 7.4 HEPES buffer or distilled water. The sample was examined using a Zeta Sizer (Model 4, Malvern Instruments, UK) (Table 1). The heat of interaction produced when DNA interacted with chitosan was measured by Isothermal Titration Microcalorimetry using a Thermal Activity Monitor (TAM, Thermometric, Model 2277, Sweden). The microcalorimetry studies were carried out by slowly adding chitosan solutions (0.05 - 0.4%) at a rate of 10 µl per 11 min, a total of 35 injections from a precision syringe to pCAT-DNA solution (2 ml; 40.5 µg/ml). As a control the appropriate chitosan solution was added to water. Each addition of titrant led to the evolution (exothermic process) or absorption (endothermic process) of heat. The heat, which was conducted through thermopiles, was recorded as a series of peaks. The value obtained for the control was used for subtracting the heat of dilution.

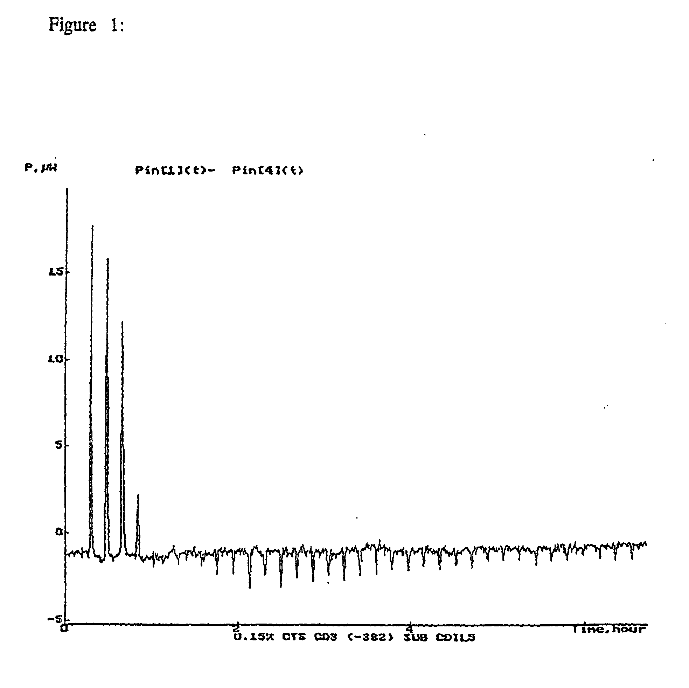

The calibrated heat flow graph of pCAT-DNA titrated with chitosan showed two groups of heat exchange (Figure 1). The first group is exothermic and the second is endothermic. The first group is from the ionic interaction between positively charged chitosan and negatively charged pCAT-DNA, since the ratio of pCAT-DNA to chitosan for the first group is 0.79 ± 0.10 (w/w) which is very close to the ratio of pCAT-DNA to chitosan when their opposite charge ratio unit is 1:1. The second group is from the change of complex conformation.

A fluorimetric method was used to provide a rapid method to characterise the condensation of pCAT-DNA by cationic polymers. This was carried out by adding ethidium bromide (EtBr) to water in a cuvette (reference) and adding pCAT-DNA solution and ethidium bromide to water in a second cuvette (test). The fluorescence of the reference cuvette was set to zero using Perkin-Elmer 3000 Fluorescence Spectrometer. The optical emission wavelength was 591 nm and optical excitation wavelength was 366 nm. The fluorescence obtained for the test cuvette was measured. Chitosan samples (C-110 and C-210) were then introduced in very small portions to the test cuvette with constant stirring, and the fluorescence reading was taken three times, returning to the zero cell in between each reading. The average of the three readings was then recorded. 8µg of pCAT-DNA were employed in each compaction study. The compaction of DNA caused by chitosan was compared with that produced by a standard cationic polymer (Poly-L-Lysine).

The pCAT chitosan complexes with ratios 1:5 and 1:10 were both positively charged (Table 1). The complexes with the lower pCAT-DNA to chitosan ration (1:10) ratio were observed to be more positively charged than the complexes with the corresponding higher ratio (1:5). This observation was as expected because chitosan is a cationic polymer, indeed a progressive increase in zeta potential (from negative to positive) was found with increase in chitosan added.

### Example 3

### Cell transfection studies

The cell lines used were Mouse Leukaemic monocyte - macrophage (RAW 264) and Rat glial tumour (C6). The RAW 264 cells were grown in Dulbecco's Modified Eagle's Medium containing 10% foetal bovine serum (FBS) and 2 mM glutamine, while the C6 cells were grown in Ham's F12 medium, containing 10% FBS and 2 mM glutamine. Both the cell lines were grown in 25 ml of the appropriate medium in 75 cm² Falcon flasks at 37°C and 5% CO₂. After the cells became subconfluent they were passaged.

### Sample preparation

The samples evaluated in these studies with respect to their ability to transfect RAW 264 and C6 cell lines were prepared as follows:
1. aqueous pCAT DNA (2 µg) was made up to 1 ml with OptiMEM.
2. aqueous pCAT DNA/ lipofectamine (LF) complex:
   - C6 cell line: 20 µl of OptiMEM containing 2 µg DNA was mixed with 20 µl of OptiMEM containing 1.2 µg LF and incubated at room temperature for 30 minutes to allow complex formation. The volume was adjusted to 160 µl with OptiMEM just prior to transfection.
   - RAW cell line: 20 µl of OptiMEM containing 2 µg DNA was mixed with 100 µl of OptiMEM containing 10 µg LF and incubated at room temperature for 30 minutes to allow complex formation. The volume was adjusted to 1 ml with OptiMEM just prior to transfection.
3. pCAT DNA complexes were resuspended in 1 ml of full culture media (without antibiotics).
4. As a control, culture media was tested in the cells.

The amounts of the four chitosan complex formulations used in the transfection studies are shown in Table 2. All the samples were tested in quadruplicate in each cell line using the procedure briefly described here. The cells were grown in 25 ml of medium to sub-confluence in a 75 cm² Falcon flask as described above. Each well on a 24 well culture plate was seeded with 1 ml (5 x 10⁵ cells/ml) of the cell suspension in full culture media and incubated overnight at 37°C, 5% CO₂ to become approximately 80% confluent. After aspirating the medium, the transfection substrates were slowly added to the cell monolayer and incubated at 37°C, 5% CO₂ for 5 hours. When transfecting the cells with microparticles the plate was agitated every 30 minutes. The transfection was terminated by replacing the transfection substrate with 1 ml of full culture media. When using microparticles the cells were thoroughly washed with PBS until the monolayer was free from particles. The plate was incubated for 48 hours prior to extracting the cells for CAT analysis.

**Table 2:**

| **the amounts of chitosan formulations used in the transfection studies** | | | |
|---|---|---|---|
| Cell line | Formulation (CAT-DNA chitosan ratio)¹ | Weight of microparticles | Amount of DNA administered to the cells (µg) |
| C6 and RAW 264 | 1:5, Small | 14.81 µg | 3 |
| C6 and RAW 264 | 1:5, Large | 14.81 µg | 3 |
| C6 and RAW 264 | 1:10 Small | 29.63 µg | 3 |
| C6 and RAW 264 | 1:10 Large | 29.63 µg | 3 |

| | | | |
|---|---|---|---|
| 1. As defined by particle size measurements, Small 100 - 200 nm, Large 300 - 500 nm | | | |

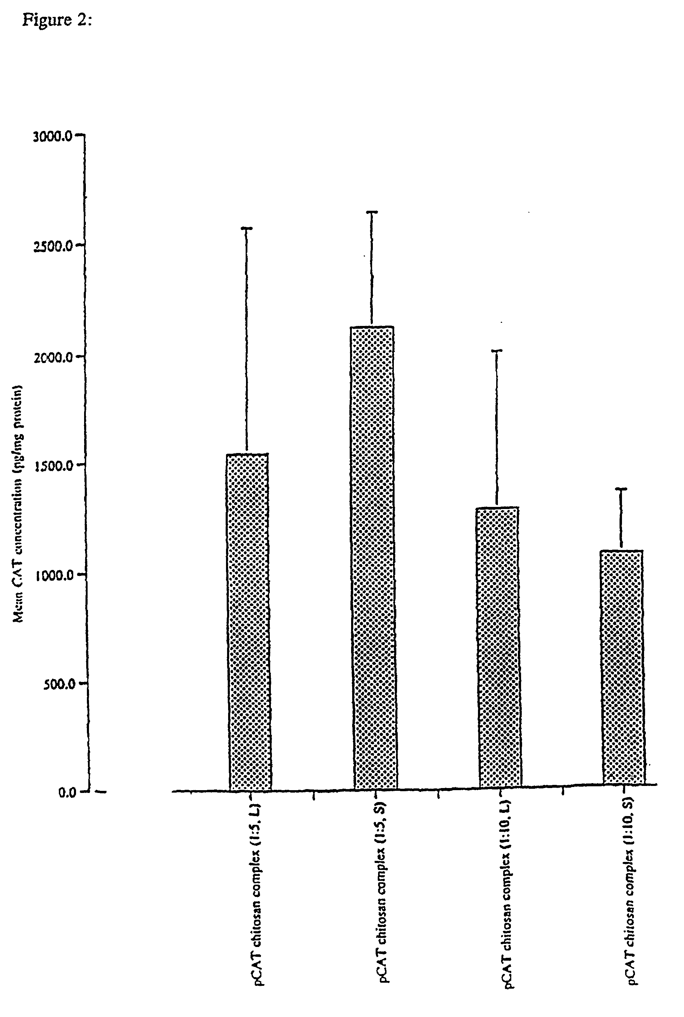

The results shown in Figure 2 showed that all the chitosan-DNA complexes transfected the cells. There was no differences between the different systems.

### Example 4: Animal studies

The administration of plasmid DNA formulations to the gastrointestinal tract of rabbits, involved laparotomy (surgical incision in the abdomen) and recovery of the rabbits. Prior to each study day fur was removed from the abdominal area of the appropriate rabbit using hair clippers. On each study day, the appropriate rabbits were anaesthetised using a Boyles Apparatus / halothane anaesthesia. Anaesthesia was maintained through the procedure. A midline incision of between 5 - 8 cm was made through the skin to expose the abdomen. A second midline incision of between 5 - 8 cm was made through the white-line (to minimise operative bleeding) running longitudinally down the abdomen of the rabbit to access the abdominal cavity. The intestines were exposed and the colon and upper part of the small intestine was identified with minimal handling of the intestines. It was necessary to exteriorise part of the intestines to gain clear access to the small intestine and colon for dose administration.

For administration to the small intestine, the dose was administered to the upper part of the small intestine approximately 10 cm from the junction with the stomach. For administration to the colon, the dose was administered to the colon approximately 5 cm from the junction with the caecum.

For dose administration, the appropriate dose volume was administered directly into the lumen of the appropriate part of the gastrointestinal tract using a sterile syringe and needle. On withdrawal of the needle the tissue was sealed with a drop of cyanoacrylate adhesive to prevent dose leakage. Care was taken to ensure that the cyanoacrylate adhesive dried before the exteriorised intestines were replaced in the abdominal cavity and that the intestines lay in approximately their natural position. The abdominal cavity was closed with two layers of sutures, one through the abdominal musculature and the second through the skin. The rabbits were returned to the appropriate housing room and allowed to recover from the effects of the anaesthetic.

Approximately seventy-two or forty eight hours after the dose administration of the formulations the rabbits were killed by an overdose of pentobarbitone sodium (approximately 0.5 ml/kg) administered via a marginal ear vein. A midline incision was made in the abdomen to expose the contents of the abdominal cavity. The alimentary canal, from the small intestines to the rectum, was removed from the abdominal cavity by cutting through the small intestine close to the junction with the stomach and through the rectum close to the anus.

### Collection of tissues from the small intestine

The small intestine from the junction with the stomach to the caecum, was categorised into three regions of approximately equal lengths which were described as anterior, medial and posterior segments. For each rabbit dosed into the small intestine a total of 8 tissue samples was collected:
- **Mesenteric lymph nodes (MLN):** These lymph nodes were aggregated forming a compact mass covering the left side of the superior mesenteric artery. These aggregated lymph nodes were collected in one sample.
- **Peyer's patches (PP):** These were collected from the small intestine as follows: 1 patch closest to site of dosing (PP1), 1 patch in medial segment (PP2), 1 patch in posterior segment (PP3). Each Peyer's patch was collected by first removing the relevant section of intestine prior to isolating the Peyer's patch.
- Enterocytes (ENT): Intestinal tissue was collected ensuring it was devoid of a Peyer's patch. A section of intestine was collected near to the site of collection of the first Peyer's patch (ENT1), in the medial segment (ENT2) and in the posterior segment (ENT3).
   From each section of intestine, a tissue sample was isolated which had a cross-sectional area approximately equal to that occupied by a Peyer's patch.
- **Colonic tissue (COL):** A segment of tissue was removed from the rectum and a tissue sample was isolated which has a cross-sectional area approximately equal to that occupied by a Peyer's patch.

### Collection of tissues from the colon

The colon, from the junction with the caecum to the rectum, was categorised into three regions of approximately equal lengths which were described as anterior and medial segments and rectum. From each rabbit dosed into the colon a total of 4 tissue samples were collected:
- **Mesenteric lymph nodes (MLN):** These aggregated lymph nodes were collected in one sample.
- **Colonic tissue (COL):** A segment of tissue was removed from the anterior of the colon close to the site of dosing (COL1), from the medial segment (COL2) and the rectum (COL3). For each segment, a tissue sample was isolated which had a cross-sectional area approximately equal to that occupied by a Peyer's patch.

Following the removal of the tissue samples from each rabbit, instruments were rinsed with sterile 0.9% sodium chloride to avoid cross-contamination. After removal, the tissue samples were washed thoroughly with 0.9% sodium chloride and placed in a petri dish. Each tissue sample was divided into two samples of approximately equal size using a sterile scalpel blade and each of these were further cut into 3-4 pieces. Pieces from each tissue sample were placed in a sterilized screw-top tube (1.5 ml) containing approximately 250 µl of 2.5 mm silica zirconium beads and promptly frozen in liquid nitrogen. The samples were stored on dry-ice prior to storage at -80°C for CAT analysis.

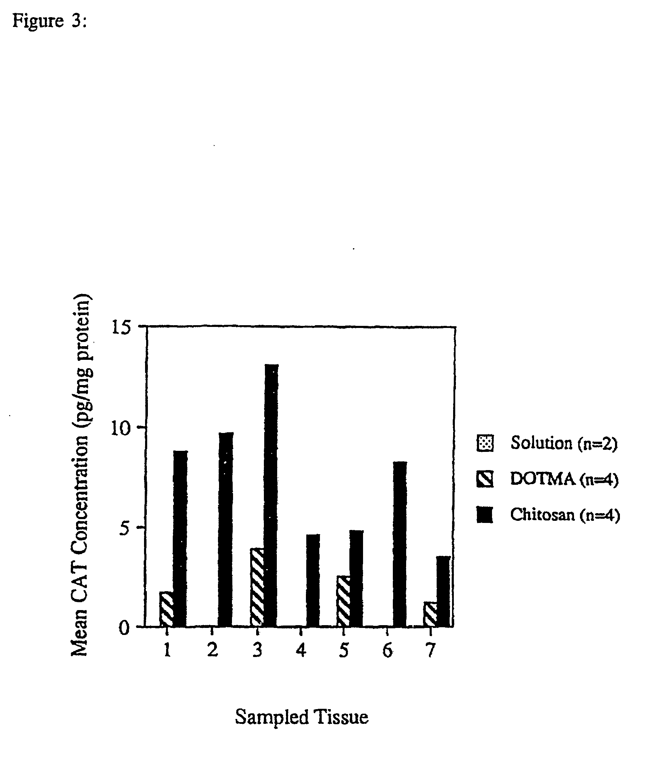

Details of the results obtained from the different formulations studied, namely the simple pCAT-DNA solution, pCAT-DNA formulated with DOTMA and the chitosan-DNA complex are given in Table 3. It can be seen that the control solution gave no expression in any of the tissues studied. The pCAT-DNA DOTMA formulation gave some expression but not in all tissues. Surprisingly the chitosan-DNA complex gave expression in almost all tissues examined. The mean data are compared in Figure 3. The surprising effect seen with the chitosan complex is well demonstrated.

**Table 3:**

| **Summary of CAT concentration of tissue extracts from rabbits dosed in the gastrointestinal tract with pCAT-DNA at 100 µg per animal (mean values)** | | | | |
|---|---|---|---|---|
| Formulation constituents | Colon sample | CAT concentration pg/mg protein | Small intestine sample | CAT concentration pg/mg protein |
| pCAT solution | COL 2 | 0.000 | PP3 | 0.000 |
| | MLN | 0.000 | ENT1 | 0.000 |
| | | | MLN | 0.000 |
| pCAT:DOTMA | COL1 | 0.000 | PP1 | 1.687 |
| | COL2 | 0.000 | PP2 | 0.000 |
| | COL3 | 0.000 | PP3 | 3.919 |
| | MLN | 0.000 | ENT1 | 2.533 |
| | | | ENT2 | 0.000 |
| | | | ENT3 | 0.000 |
| | | | Col | 1.185 |
| | | | MLN | 1.151 |
| pCAT:Chitosan | COL1 | 1.046 | PP1 | 8.086 |
| | COL2 | 0.000 | PP2 | 9.700 |
| | COL3 | 0.000 | PP3 | 13.143 |
| | MLN | 2.170 | ENT1 | 4.605 |
| | | | ENT2 | 4.824 |
| | | | ENT3 | 8.274 |
| | | | COL | 1.652 |
| | | | MLN | 0.000 |

### Example 6: Control Study

The importance of particle size of the chitosan system in providing good transfection both in vitro and in vivo was determined using chitosan microspheres prepared by a spray drying process using a 0.5% solution of Chitosan CL 113 in acetic acid. A LabPlant SD05 spray dryer was used. pCAT-DNA was adsorbed to the surface of these particles by addition of 10% aqueous suspension of microspheres into the pCAT-DNA solution with constant stirring. An adsorption efficiency of 97% was obtained. The concentration of pCAT-DNA in the formulation was 47.8 µg/ml. The transfection of the C6 cell line was conducted as in Example 2. This represented about 1 µg of DNA administered to the cell. The mean CAT concentration found was approximately 100 pg/mg protein using 0.3 mg of particles. These microspheres had a mean particle size (mean volume diameter) of 5.5 micron. The animal studies conducted as in Example 3 using 4 animals per group for both intestinal and colonic administration showed lower levels of expression than for the Chitosan-DNA complexes.

### Example 7: Evaluation of chitosan-pCAT DNA complex following nasal administration in rats

In this study, plasmid DNA construct (pCAT DNA) that carries the reporter gene for chloramphenicol acetyl transferase (CAT) and containing a cytomegalovirus promoter was used. Chitosan-pCAT DNA and Lipofectin® -pCAT DNA complexes in liquid formulations were evaluated following nasal administration in rats.

### (1) Preparation of chitosan-pCAT DNA complex

1A Chitosan-pCAT DNA complex was prepared by adding 1 ml chitosan (4 mg/ml) to 10 ml pCAT DNA solution (40 µg/ml) while stirring on a magnetic stirrer.
1B 1% mannitol (0.4 ml) solution was added to the Chitosan-pCAT DNA complex and freeze dried.
1C The freeze dried complex was resuspended in 0.8 ml ultrapure water. The final concentration of pCAT DNA was 0.5 mg/ml and that of chitosan was 5 mg/ml in the formulation (i.e. DNA:chitosan = 1:10).

### (2) Preparation of Lipofectin®-DNA complex

2A Lipofectin® (350 µl, 350 µg) was added dropwise to 220 µl pCAT DNA (175 µg) while stirring.
2B The mixture was incubated at room temperature for 15 min.
2C The final concentration pCAT DNA was 307 µl/ml and that of Lipofectin® was 614 µg/ml (ie DNA:Lipofectin® = 1:2).

### (3) Rat Studies

3A The two formulations prepared as described above (sections 1 and 2) were administered intranasally to groups of male Wistar rats as follows:

| | |
|---|---|
| Group 1 | 100 µl (50 µl per nostril) chitosan-pCAT DNA complex administered to three rats; (pCAT DNA dose = 100 µg) |
| Group 2 | 100 µl (50 µl per nostril) Lipofectin® -pCAT DNA complex administered to five rats; (pCAT DNA dose = 61.4 µg) |

3B Prior to dose administration, rats were sedated with 0.4 ml/kg Hypnorm administered intramuscularly. This was intended for animal immobilisation and to reduce stress in animals during dosing. Maintenance of animal immobilisation for 1-2 hours would allow adequate contact time between the liquid doses and the nasal membrane. The animals were kept warm (approximately 38°C) throughout the procedure.
3C Following sedation the animals were dosed with 50 µl of the formulation first into the left nostril and after five minutes into the right nostril using a needle and a Hamilton syringe attached to a suitable length of polythene tubing. The animals with their ventral surface up were allowed to remain on a heated table for 60 min.

### (4) Removal of rat tissues

Approximately 72 hours after dosing, the animals were killed by an overdose of pentobarbitone sodium administered intravenously. The nasal mucosal tissues, both right and left were removed followed by mandibular lymph nodes, oesophagus and the lungs. Each tissue sample was rinsed with isotonic potassium chloride solution and immediately froze in liquid nitrogen. The samples were stored at -80°C.

### (5) Extraction of tissues

5A Prior to extraction the tissues were rapidly thawed and transferred to tubes containing 300 µl Zirconium/ silica beads (2.5 mm). The tissues were homogenised in 0.5 ml extraction buffer (10 mM Tris pH 7.8, 150 mM sodium chloride, 1 mM EDTA, 0.5% Triton X-100, 1.0 µM pepstatin, 100 µM Leupeptin and 0.25 mM PMSF) using a Mini bead beater (5 cycles of 30 sec homogenization and 30 sec rest).
5B The homogenates were transferred to microfuge tubes and centrifuged at 13 000 rpm for 20 min at 4°C. The supernatants were transferred to microfuge tubes and temporarily stored on ice.

### (6) Protein analysis

The tissue extracts were diluted 1 in 20 with phosphate buffered saline and the protein concentration was determined using the Pierce BCA protein assay. The total protein concentration in each tissue extract was calculated after correcting for dilution.

### (7) CAT analysis

7A On the day of preparation, the tissue extracts were appropriately diluted with the sample buffer supplied in the CAT ELISA kit (suppliers, Boehringer Mannheim). The diluted samples were stored at -80°C.
7B On the day of analysis the sample dilutions were rapidly thawed and analysis for CAT ELISA kit following the protocol provided by the suppliers.
7C The total CAT concentration in each tissue extract was calculated after correcting for dilution (Table 4).
7D The amount of CAT expression in each tissue was then represented as pg CAT per mg protein.

### (8) Results

8A The results show that after nasal administration of chitosan-pCAT DNA, CAT is expressed in all the tissues analysed. The mandibular lymph nodes and the nasal mucosal tissues appear to express CAT better than the oesophagus and the lungs.
8B Although CAT expression in the lungs is not very high when the formulation is administered intranasally, the results indicate that the lungs have the potential for being transfected with DNA.
8C Lipofectin®-pCAT DNA formulation was expected to give effective transfection. However, it was found that the nasal administration of this formulation resulted in very little CAT expression in the various tissues. There was only some indication of CAT expression in the oesophagus and the lung tissue. This was possibly because the ratio of Lipofectin® to DNA was very low (ie 2:1).

### (9) Conclusions

9A It can be concluded from this study that the nasal route has the potential for DNA delivery.
9B Although it was not possible to estimate the amount of formulation that reached the lungs in this study, the expression of CAT on the lung tissues indicate that there is potential for pulmonary delivery of DNA via the nasal route or possibly directly via the pulmonary route.

**Table 4:**

| **CAT concentration of tissue extracts from rats dosed intranasally with Chitisan-pCAT DNA at 100 µg or Lipofectin®-pCAT DNA at 61.4 µg pCAT DNA per animal** | | | | |
|---|---|---|---|---|
| | **CAT concentration (pg/mg protein)** | | | |
| | **Chitosan-pCAT DNA (Group 1)** | | **Lipofectin® -pCATDNA (Group 2)** | |
| **Sample** | **Mean** | **SD** | **Mean** | **SD** |
| Nasal mucosal tissue | 9.38 | 8.39 | 0.00 | 0.00 |
| Mandibular lymph node | 13.72 | 21.24 | 0.00 | 0.00 |
| Oesophagus | 4.22 | 7.31 | 2.55 | 3.37 |
| Right lung | 4.32 | 4.95 | 0.37 | 0.82 |
| Left lung | 2.36 | 0.91 | 0.00 | 0.00 |

### Example 8

A DNA plasmid chitosan complex with a ratio of DNA to chitosan of 1:10 is prepared by the slow addition of chitosan (CL113, Pronova Biopolymer) solution (4 mg/ml) to a solution of DNA (4 µl/ml), DNA plasmid containing Fragment C of tetanus toxin under the control of the human cytomegaloviras major intermediate early (Anderson *et al,* Vaccine, **15,** 827 (1997)) as outlined in Example 2. The preparation is controlled to obtain particles of a size in the range 100 to 300 nm. The particles are freeze dried after the addition of 5% mannitol.

The freeze dried complex is dispersed in water immediately prior to administration and if necessary for tonicity purposes an additional amount of mannitol may be added. The concentration of the DNA complex is 100 µg/100 µl. The suspension is filled in a Monospray (Valois) nasal spray system and administered to the nasal cavity of the patient in a dose of 100 µl. The administration can, if needed, be repeated after 14 days.

## Claims

1. Use of a composition comprising a particulate complex of chitosan and nucleic acid wherein the complex is between 10 nm and 1µm in size and carries a surface charge in the manufacture of a medicament for the delivery of said nucleic acid to an epithelial surface of a mammal, wherein the nucleic acid is capable of being expressed and encodes an antiinflammatory agent, an antiinfective agent, sucrose-isomaltase, lactase-phlorizin hydrolase, maltase-glucoamylase, a drug or an antisense agent for the treatment of colon cancer.

2. A use according to Claim 1, provided that, when the nucleic acid is DNA, the surface charge of the composition is above +10 mV for DNA:chitosan ratios between 1:1 and 1:6 and above +20mV for all other DNA:chitosan ratios.

3. A use according to Claim 1 or Claim 2 wherein the nucleic acid is DNA.

4. A use according to any one of Claims 1 to 3 where the nucleic acid encodes an antiinflammatory agent.

5. A use according to any one of Claims 1 to 3 wherein the nucleic acid encodes an antiinfective agent.

6. A use according to any one of Claims 1 to 3 wherein the nucleic acid encodes sucrose-isomaltase, lactase-phlorizin hydrolase or maltase-glucoamylase.

7. A use according to any one of Claims 1 to 3 wherein the nucleic acid encodes a drug or antisense agent for the treatment of colon cancer.

8. A use according to any one of Claims 1 to 7 wherein the complex is bound to the surface of a larger microsphere.

9. A use according to any one of Claims 1 to 8 wherein the epithelial surface is the gastrointestinal tract, the small intestine, the colon, the gut associated lymphoid tissue, the nasal mucosa, the nose associated lymphoid tissue, the vagina, the buccal cavity, the oesophegus, the rectum, the eye, or the inner ear, or comprises a region of the lung.

10. A use according to Claim 9 wherein a mucolytic agent, lectin, adhesin or invasin is to be coadministered.

11. Use according to Claim 6 in the treatment of disaccharide intolerance.

## Patentansprüche

1. Verwendung einer Zusammensetzung, die einen partikulären Komplex von Chitosan und Nukleinsäure umfaßt, wobei der Komplex eine Größe zwischen 10 nm und 1 µm besitzt und eine Oberflächenladung aufweist, bei der Herstellung eines Medikamentes für die Abgabe der Nukleinsäure an eine epitheliale Oberfläche eines Säugetiers oder Menschen, wobei die Nukleinsäure explimiert werden kann und einen antientzündlichen Wirkstoff, einen antiinfektiösen Wirkstoff Sucrose-Isomaltase, Laktase-Phlorizinhydrolase, Maltase-Glukoamylase, ein Medikament oder einen gegensinnigen Wirkstoff für die Behandlung von Dickdarmkrebs kodiert.

2. Verwendung nach Anspruch 1, bei der unter der Voraussetzung, daß die Nukleinsäure DNA ist, die Oberflächenladung der Zusammensetzung oberhalb von +10 mV für DNA/Chitosan-Verhältnisse zwischen 1:1 und 1:6 und oberhalb von +20 mV für alle anderen DNA/Chitosan-Verhältnisse liegt.

3. Verwendung nach Anspruch 1 oder 2, bei der die Nukleinsäure DNA ist.

4. Verwendung nach einem der Ansprüche 1 bis 3, bei der die Nukleinsäure einen anti-entzündlichen Wirkstoff kodiert.

5. Verwendung nach einem der Ansprüche 1 bis 3, bei der die Nukleinsäure einen anti-infektiven Wirkstoff kodiert.

6. Verwendung nach einem der Ansprüche 1 bis 3, bei der die Nukleinsäure Sucrose-lsomaltase, Laktase-Phlorizinhydrolase oder Maltase-Glukoamylase kodiert.

7. Verwendung nach einem der Ansprüche 1 bis 3, bei der die Nukleinsäure ein Medikament oder einen gegensinnigen Wirkstoff für die Behandlung von Dickdarmkrebs kodiert.

8. Verwendung nach einem der Ansprüche 1 bis 7, bei der der Komplex an die Oberfläche eines größeren Mikrokügelchens gebunden ist.

9. Verwendung nach einem der Ansprüche 1 bis 8, bei der die epitheliale Oberfläche der Magen-Darm-Trakt, der Dünndarm, der Dickdarm, das dem Darm zugeordnete lymphoide Gewebe, die Nasenschleimhaut, das der Nase zugeordnete lymphoide Gewebe, die Vagina, der Rachenhohlraum, die Speiseröhre, das Rektum, das Auge oder das Innenohr ist oder einen Bereich der Lunge umfaßt.

10. Verwendung nach Anspruch 9, bei der ein mukolytischer Wirkstoff, Lektin, Adhesin oder Invasin mit verabreicht werden soll.

11. Verwendung nach Anspruch 6 bei der Behandlung von Dissaccharid Intoleranz.

## Revendications

1. Utilisation d'une composition comprenant un complexe particulaire de chitosane et d'acide nucléique dans lequel le complexe a une taille comprise entre 10 nm et 1 µm et porte une charge de surface dans la fabrication d'un médicament pour la délivrance dudit acide nucléique sur une surface épithéliale de mammifère, dans laquelle l'acide nucléique est capable d'être exprimé et code pour un agent antiinflammatoire, un agent antiinfectieux, la sucrose-isomaltase, la lactase-phlorizine hydrolase, la maltase-glucoamylase, un médicament ou un agent antisens pour le traitement du cancer du colon.

2. Utilisation selon la revendication 1, pourvu que, quand l'acide nucléique est de l'ADN, la charge de surface de la composition soit supérieure à +10 mV pour les rapports d'ADN:chitosane compris entre 1:1 et 1:6 et au-dessus de +20 mV pour tous les autres rapports ADN:chitosan.

3. Utilisation selon la revendication 1 ou la revendication 2 dans laquelle l'acide nucléique est un ADN.

4. Utilisation selon l'une quelconque des revendications 1 à 3 dans laquelle l'acide nucléique code pour un agent antiinflammatoire.

5. Utilisation selon l'une quelconque des revendications 1 à 3 dans laquelle l'acide nucléique code pour un agent antiinfectieux.

6. Utilisation selon l'une quelconque des revendications 1 à 3 dans laquelle l'acide nucléique code pour la sucrose-isomaltase, la lactase-phlorizine hydrolase ou la maltase-isoamylase.

7. Utilisation selon l'une quelconque des revendications 1 à 3 dans laquelle l'acide nucléique code pour un médicament ou un agent antisens pour le traitement du cancer du colon.

8. Utilisation selon l'une quelconque des revendications 1 à 7 dans laquelle le complexe est fixé à la surface d'une microsphère plus grande.

9. Utilisation selon l'une quelconque des revendications 1 à 8 dans laquelle la surface épithéliale est le tractus gastro-intestinal, le petit intestin, le colon, le tissu lymphoïde associé à l'intestin, la muqueuse nasale, le tissu lymphoïde associé au nez, le vagin, la cavité buccale, l'oesophage, le rectum, l'oeil, ou l'oreille interne, ou comprend une région du poumon.

10. Utilisation selon la revendication 9 dans laquelle un agent mucolytique, une lectine, une adhésine ou l'invasine doit être co-administré.

11. Utilisation selon la revendication 6 dans le traitement de l'intolérance aux disaccharides.
